# EUROPEAN PATENT APPLICATION

(11) **EP 3 831 399 A1**
(43) Date of publication of application: **09.06.2021**
(21) Application number: 19844221.2
(22) Date of filing: 25.07.2019
(51) Int. Cl.: A61K 36/05, A01N 43/16, A01N 65/03, A01P 1/00, A23K 10/16, A23L 33/105, A23L 33/12, A61K 31/7032, A61K 35/68, A61K 35/748, A61K 36/02, A61P 31/14

(54) **ANTI-NOROVIRUS AGENT**

(30) Priority: 31.07.2018 JP 2018143050
(71) Applicant: DENSO CORPORATION, Kariya-city, Aichi 448-8661 (JP); Chubu University Educational Foundation, Kasugai-shi Aichi 487-8501 (JP)
(72) Inventor: ASANO Mana, Kariya- city, Aichi 4488661 (JP); KUNO Hitoshi, Kariya- city, Aichi 4488661 (JP); ATSUMI Kinya, Kariya- city, Aichi 4488661 (JP); HAYASHI Kyoko, Kasugai-shi, Aichi 487-8501 (JP); KAWAHARA Tosihio, Kasugai-shi, Aichi 487-8501 (JP); KOMATSU Satoko, Kariya- city, Aichi 4488661 (JP)
(74) Representative: Kuhnen & Wacker Patent- und Rechtsanwaltsbüro PartG mbB
(86) International application number: PCT/JP2019/029306
(87) International publication number: WO 2020/026951

(57) **Abstract**

An object of the invention is to provide a novel means effective for treatment of infectious diseases due to norovirus or prevention of spreading of infectious diseases due to norovirus. An anti-norovirus agent containing a unicellular alga-derived substance as an effective ingredient is provided.

## Description

### TECHNICAL FIELD

The present invention relates to an anti-norovirus agent. Specifically, the invention relates to an anti-norovirus agent using a substance derived from a microalga, use application thereof, and the like. This application claims the benefit of priority from prior Japanese Patent Application No. 2018-143050 filed on July 31, 2018, the entire contents of which are hereby incorporated by reference.

### BACKGROUND ART

Noroviruses are non-enveloped single-stranded positive-sense RNA viruses and are classified into the genus Norovirus in the family Caliciviridae. In recent years, noroviruses become a main cause of infectious gastroenteritis, have extremely strong infectability, exhibit high resistivity with respect to surfactants, alcohols, or the like, and often cause mass infection. Clams such as oysters are main infectious sources, but secondary infection from vomited materials or excrements of patients becomes a serious problem.

It is a current situation that norovirus infection is coped with by heating food, sterilizing cooking devices, sterilizing fingertips, and the like; on the other hand, an antivirus agent or a virucidal agent targeting noroviruses has been proposed (for example, see Patent Documents 1 to 3). Incidentally, until now, artificial culturing of noroviruses has not achieved success, and this becomes a barrier of development of vaccines or therapeutic agents.

### PRIOR ART LITERATURE

### Patent Documents

Patent Document 1: JP 2013-47196 A
Patent Document 2: JP 6139813 B1
Patent Document 3: JP 2009-292736 A

### SUMMARY OF THE INVENTION

### PROBLEM TO BE SOLVED BY THE INVENTION

Under the above circumstances, an object of the invention of the present application is to provide a novel means effective for treatment of infectious diseases due to norovirus (infectious gastroenteritis) and/or prevention of spreading of infectious diseases due to norovirus.

### MEANS FOR SOLVING THE PROBLEMS

One of features of the norovirus infectious diseases is egestion of an infectious virus into the feces of a patient over several days to approximately one week even after the symptom is not recognized, and this causes secondary infection spread. The studies have been conducted with attention to this point, and as a result, it has been found that an alga body of a microalga of the genus Coccomyxa and an extract thereof (a monogalactosyldiacylglycerol-containing extract) has an action of promoting egestion/elimination of the virus from the body in addition to an action of suppressing norovirus proliferation, and is extremely effective in treatment of norovirus infectious diseases and prevention and inhibition of secondary infection. Furthermore, the important point is that the above-described actions are recognized even at the time of diminishing the immune function. This situation indicates that the alga body of the microalga of the genus Coccomyxa and the extract thereof are extremely effective as effective ingredients of therapeutic agents of norovirus infectious diseases. Meanwhile, by further studies, the structure of monogalactosyldiacylglycerol that is an effective ingredient in the extract has become clear. Furthermore, further knowledge supporting the fact that the usefulness of the extract is extremely high has been obtained.

On the other hand, it has become clear that the same effect as that of the extract containing monogalactosyldiacylglycerol derived from the microalga of the genus Coccomyxa can be expected also in extracts containing monogalactosyldiacylglycerol derived from Chlorella vulgaris, Nannochloropsis oculata, Arthrospira platensis (spirulina), and Euglena gracilis.

The following inventions are provided mainly based on the above-described achievement and discussion.
[1] An anti-norovirus agent including a substance derived from a unicellular alga as an effective ingredient.
[2] The anti-norovirus agent described in [1], in which the unicellular alga is a microalga belonging to the genus Coccomyxa, Chlorella, Nannochloropsis, Arthrospira, or Euglena.
[3] The anti-norovirus agent described in [1], in which the unicellular alga is a microalga belonging to the genus Coccomyxa.
[4] The anti-norovirus agent described in [3], in which the microalga is a coccomyxa sp. KJ strain or a mutant strain thereof.
[5] The anti-norovirus agent described in any one of [1] to [4], in which the effective ingredient is a monogalactosyldiacylglycerol-containing extract of the unicellular alga.
[6] The anti-norovirus agent described in [5], in which a primary ingredient is monogalactosyldiacylglycerol.
[7] The anti-norovirus agent described in [6], in which the monogalactosyldiacylglycerol is one or more of monogalactosyldiacylglycerols selected from the group consisting of the following (1) to (6):
   (1) monogalactosyldiacylglycerol in which a constituent fatty acid is C16:3 and C18:3;
   (2) monogalactosyldiacylglycerol in which a constituent fatty acid is C16:2 and C18:3;
   (3) monogalactosyldiacylglycerol in which a constituent fatty acid is C18:3 and C18:3;
   (4) monogalactosyldiacylglycerol in which a constituent fatty acid is C16:2 and C18:2;
   (5) monogalactosyldiacylglycerol in which a constituent fatty acid is C18:3 and C18:2; and
   (6) monogalactosyldiacylglycerol in which a constituent fatty acid is C16:1 and C18:2.
[8] The anti-norovirus agent described in any one of [5] to [7], in which the effective ingredient is a substance obtained by subjecting an ethanol extract of the unicellular alga to chromatographic refinement and increasing a content of monogalactosyldiacylglycerol.
[9] The anti-norovirus agent described in any one of [5] to [8], in which a monogalactosyldiacylglycerol content in the effective ingredient is 70% (w/v) to 99% (w/v).
[10] The anti-norovirus agent described in any one of [1] to [4], in which the effective ingredient is an alga body.
[11] The anti-norovirus agent described in any one of [1] to [4], in which the effective ingredient is dry powder of an alga body.
[12] An anti-norovirus agent containing, as an effective ingredient, one or more of monogalactosyldiacylglycerols selected from the group consisting of the following (1) to (6):
   (1) monogalactosyldiacylglycerol in which a constituent fatty acid is C16:3 and C18:3;
   (2) monogalactosyldiacylglycerol in which a constituent fatty acid is C16:2 and C18:3;
   (3) monogalactosyldiacylglycerol in which a constituent fatty acid is C18:3 and C18:3;
   (4) monogalactosyldiacylglycerol in which a constituent fatty acid is C16:2 and C18:2;
   (5) monogalactosyldiacylglycerol in which a constituent fatty acid is C18:3 and C18:2; and
   (6) monogalactosyldiacylglycerol in which a constituent fatty acid is C16:1 and C18:2.
[13] The anti-norovirus agent described in any one of [1] to [12], in which the anti-norovirus agent exhibits an action of suppressing norovirus proliferation of norovirus and/or an action of promoting egestion of the virus from the body.
[14] A composition containing the anti-norovirus agent described in any one of [1] to [13].
[15] The composition described in [14], in which the composition is a medicine for norovirus infectious diseases.
[16] The composition described in [14], in which the composition is food or feed.

### BRIEF DESCRIPTION OF THE DRAWINGS

Fig. 1 shows results of an experiment using an alga body (dry powder) of a coccomyxa sp. KJ strain. The amount of virus in the excrement after murine norovirus (MNV) infection in 5-FU untreated mice is shown.
Fig. 2 shows results of an experiment using an alga body (dry powder) of a coccomyxa sp. KJ strain. The amount of virus of 10 to 21 days after MNV infection in 5-FU untreated mice is shown.
Fig. 3 shows results of an experiment using an alga body (dry powder) of a coccomyxa sp. KJ strain. The amount of virus in the excrement after MNV infection in 5-FU treated mice is shown.
Fig. 4 shows results of an experiment using an alga body (dry powder) of a coccomyxa sp. KJ strain. The amount of virus of 10 to 21 days after MNV infection in 5-FU treated mice is shown.
Fig. 5 shows results of an experiment using an alga body (dry powder) of a coccomyxa sp. KJ strain. Comparison is made regarding the days elapsed to the virus elimination.
Fig. 6 shows results of an experiment using an alga body (dry powder) of a coccomyxa sp. KJ strain. The neutralizing antibody titers in the blood serum of 21 days after infection were compared and evaluated. ** p < 0.01 vs. 5-FU untreated control group, ## p < 0.01 vs. 5-FU treated control group
Fig. 7 shows results of an experiment using an MGDG preparation of a coccomyxa sp. KJ strain. The amount of virus in the excrement after MNV infection in 5-FU untreated mice is shown.
Fig. 8 shows results of an experiment using an MGDG preparation of a coccomyxa sp. KJ strain. The amount of virus of 10 to 21 days after MNV infection in 5-FU untreated mice is shown.
Fig. 9 shows results of an experiment using an MGDG preparation of a coccomyxa sp. KJ strain. The amount of virus in the excrement after MNV infection in 5-FU treated mice is shown.
Fig. 10 shows results of an experiment using an MGDG preparation of a coccomyxa sp. KJ strain. The amount of virus of 10 to 21 days after MNV infection in 5-FU treated mice is shown.
Fig. 11 shows results of an experiment using an MGDG preparation of a coccomyxa sp. KJ strain. The elapsed days to virus elimination were compared.
Fig. 12 shows results of an experiment using an MGDG preparation of a coccomyxa sp. KJ strain. The neutralizing antibody titers in the blood serum of 21 days after infection were compared and evaluated. *** p < 0.001, ## p < 0.01 vs. control
Fig. 13 shows results of a virucidal activity test targeting feline calicivirus (FCV). A virus fluid was treated for 0-60 minutes with 10 µg/ml, 50 µg/ml, and 200 µg/ml of MGDG preparations.
Fig. 14 shows an example of a culture medium used in the culturing of a microalga of the genus Chlorella (C culture medium) and an example of a culture medium used in the culturing of a microalga of the genus Nannochloropsis (ESM culture medium).
Fig. 15 shows an example of a culture medium used in the culturing of a microalga of the genus Arthrospira (MA culture medium) and an example of a culture medium used in the culturing of an alga of the genus Euglena (HUT culture medium).
Fig. 16 shows result of a virucidal activity test in the presence of an organic material. Feline calicivirus (FCV) was used as a replacement for norovirus.
Fig. 17 shows analysis results of a component (MGDG1) contained in an MGDG preparation (chromatogram of GC/FID).
Fig. 18 shows analysis results of a component (MGDG2) contained in an MGDG preparation (chromatogram of GC/FID).
Fig. 19 shows analysis results of a component (MGDG3) contained in an MGDG preparation (chromatogram of GC/FID).
Fig. 20 shows analysis results of a component (MGDG4) contained in an MGDG preparation (chromatogram of GC/FID).
Fig. 21 shows analysis results of a component (MGDG5) contained in an MGDG preparation (chromatogram of GC/FID).
Fig. 22 shows virucidal activity of each MGDG fraction (MGDG1 to MGDG5). The virucidal activities of MGDG1 to MGDG5 and the MGDG preparation (Mix product) with respect to influenza virus, herpes simplex virus type 2, feline calicivirus, and poliovirus were compared.
Fig. 23 shows an HPLC chromatogram of MGDG preparations of various organisms (a coccomyxa sp. KJ strain, Chlorella vulgaris, Nannochloropsis oculata, and Arthrospira platensis (spirulina)). MGDG-1 to MGDG-5 are peaks derived from the KJ strain MGDG preparation, and peaks estimated as the same structure as in these peaks are indicated by *.
Fig. 24 shows an HPLC chromatogram of MGDG preparations of various organisms (a coccomyxa sp. KJ strain, Euglena gracilis (Inochino Euglena, Biozyme), and Spinacia oleracea). MGDG-1 to MGDG-5 are peaks derived from the KJ strain MGDG preparation, and peaks estimated as the same structure as in these peaks are indicated by *.

### MODES FOR CARRYING OUT THE INVENTION

### 1. Terminology and action

An anti-norovirus agent is an antivirus agent targeting noroviruses. As shown in Examples described below, an anti-norovirus agent of the invention can be expected to have an action of suppressing proliferation of noroviruses and also an action of promoting egestion (elimination) of the viruses from the body (an action of egesting the norovirus from the body in an early stage). The latter action is extremely effective and important in terms of prevention and inhibition of secondary infection.

### 2. Effective ingredient of anti-norovirus agent

The anti-norovirus agent of the invention contains a substance derived from a unicellular alga as an effective ingredient. The unicellular alga (Trebouxiophyceae, Eustigmatophyceae, Cyanophyceae, Euglenophyceae, or the like) is not particularly limited as long as anti-norovirus activity is recognized. Preferred examples of the unicellular alga include microalga of the genus Coccomyxa (as a specific example, coccomyxa sp. KJ strain), microalga of the genus Chlorella (as a specific example, Chlorella vulgaris), microalga of the genus Nannochloropsis (as a specific example, Nannochloropsis oculata), microalga of the genus Arthrospira (as a specific example, Arthrospira platensis (spirulina)), and microalga of the genus Euglena (as a specific example, Euglena gracilis). Among these, the microalga of the genus Coccomyxa is particularly preferred. The microalga of the genus Coccomyxa is not particularly limited, and as a preferred example, a coccomyxa sp. KJ strain or a mutant strain thereof can be exemplified. The coccomyxa sp. KJ strain (KJ Denso) is deposited to National Institute of Technology and Evaluation, Biotechnology Center, International Patent Organism Depositary (NITE-IPOD) (#120, 2-5-8 Kazusakamatari, Kisarazu-shi, Chiba) on June 4, 2013 under the accession number FERM P-22254, and is transferred to an international depositary authority under the Budapest Treaty on June 2, 2015 under the accession number FERM BP-22254. Incidentally, the previous genus Pseudococcomyxa is included in the genus Coccomyxa in the latest classification (reference document: PLoS One. 2015 Jun 16; 10(6): e0127838. doi: 10.1371/journal.pone.0127838. eCollection 2015.).

A mutant strain of the coccomyxa sp. KJ strain can be obtained by irradiation with ultraviolet rays, X rays, or γ rays, a mutagen treatment, multiple beam irradiation, gene manipulation (such as introduction of a foreign gene, gene disruption, and inheritable genetic modification by genome editing), and the like. The method for acquiring a mutant strain, characteristics of a mutant strain, and the like are not particularly limited as long as a mutant strain that produces monogalactosyldiacylglycerol exhibiting anti-norovirus activity is obtainable.

The method for culturing the microalga of the genus Coccomyxa is not particularly limited. As a culture medium for culturing the microalga of the genus Coccomyxa, a culture medium that is generally used in the culturing of a microalga may be used, and for example, any of a known culture medium for a freshwater microalga and a known culture medium for a marine microalga containing various nutrient salts, trace metal salts, vitamins, and the like can be used. As the culture medium, for example, an AF6 culture medium is exemplified. The composition (per 100 ml) of the AF6 culture medium is as follows.

| | |
|---|---|
| NaNO₃ | 14 mg |
| NH₄NO₃ | 2.2 mg |
| MgSO₄.7H₂O | 3 mg |
| KH₂PO₄ | 1 mg |
| K₂HPO₄ | 0.5 mg |
| CaCl₂.2H₂O | 1 mg |
| CaCO₃ | 1 mg |
| Fe-citrate | 0.2 mg |
| Citric acid | 0.2 mg |
| Biotin | 0.2 µg |
| Thiamine HCl | 1 µg |
| Vitamin B₆ | 0.1 µg |
| Vitamin B₁₂ | 0.1 µg |
| Trace metals | 0.5 mL |
| Distilled water | 99.5 mL |

Examples of the nutrient salts include nitrogen sources such as NaNO₃, KNO₃, NH₄Cl, and urea, and phosphorus sources such as K₂HPO₄, KH₂PO₄, and sodium glycerophosphate. Furthermore, examples of the trace metal include iron, magnesium, manganese, calcium, and zinc, and examples of vitamins include vitamin B1 and vitamin B12.

As for the culturing method, stirring may be performed along with supplying of carbon dioxide under a ventilation condition. At this time, culturing is performed by light irradiation for 12 hours by a fluorescent lamp, light irradiation with light-dark cycle such as a dark condition for 12 hours, or continuous light irradiation. Culture conditions are also not particularly limited insofar as not adversely affecting the proliferation of the microalga of the genus Coccomyxa, and for example, the pH of a culture solution is set to 3 to 9 and the culture temperature is set to 10°C to 35°C.

Incidentally, with respect to the method for culturing the coccomyxa sp. KJ strain, JP 2015-15918 A, WO 2015/190116 A1, Satoh, A. et al., Characterization of the Lipid Accumulation in a New Microalgal Species, Pseudochoricystis ellipsoidea (Trebouxiophyceae) J. Jpn. Inst. Energy (2010) 89:909-913., and the like will serve as a reference.

As an effective ingredient, a monogalactosyldiacylglycerol-containing extract of a unicellular alga (particularly preferably, a microalga belonging to the genus Coccomyxa) (first embodiment), an alga body, or dry powder thereof (second embodiment) is used. Hereinafter, each embodiment will be described. It is noted either one of the monogalactosyldiacylglycerol-containing extract, the alga body, or dry powder thereof is generally used as an effective ingredient, but as the effective ingredient, both of these can be used.

### (1) Monogalactosyldiacylglycerol-containing extract

In an embodiment of the invention, as an effective ingredient, a "monogalactosyldiacylglycerol-containing extract of a unicellular alga (particularly preferably, a microalga belonging to the genus Coccomyxa)" is used. An extraction method is not particularly limited as long as an extract containing monogalactosyldiacylglycerol is obtainable. As the extraction method, for example, ethanol extraction can be employed. Preferably, a substance obtained by refinement after ethanol extraction with increased content of monogalactosyldiacylglycerol is used as a "monogalactosyldiacylglycerol-containing extract of a unicellular alga (particularly preferably, a microalga belonging to the genus Coccomyxa)". Examples of the refinement method include column chromatography using a filler such as silica gel or alumina, gel filtration chromatography, and condensation.

Before the extraction, the recovered alga body may be subjected to a dry treatment and/or a crushing treatment. In other words, a dried alga body, a dried or crushed alga body (typically, dry powder), or a crushed alga body may be prepared, and an extraction operation may be performed by using this alga body. A unicellular alga subjected to a processing treatment in advance (for example, a dried alga body, powder/pulverulent body thereof, a tablet (in which an excipient or the like may be contained), or the like) may be purchased and subjected to the extraction operation. For example, as for Chlorella vulgaris, Nannochloropsis oculata, Arthrospira platensis (spirulina), Euglena gracilis, and the like, processed products thereof are commercially available.

The content of monogalactosyldiacylglycerol in the effective ingredient is not particularly limited as long as the extract exhibits anti-norovirus activity, and the content thereof is, for example, 70% (w/v) to 99% (w/v), preferably 80% (w/v) to 99% (w/v), further preferably 90% (w/v) to 99% (w/v), and even more preferably 95% (w/v) to 99% (w/v) (as specific examples, 96% (w/v), 97% (w/v), and 98% (w/v)). In principle, as the content of monogalactosyldiacylglycerol is increased, strong anti-norovirus activity can be expected. The "monogalactosyldiacylglycerol" is one of glycoglycerolipids and is known as a constituent of chloroplast thylakoid membranes of plants. The monogalactosyldiacylglycerol has a skeleton in which galactose is bound to glycerol by β-linkage.

Preferably, in the anti-norovirus agent of the invention, monogalactosyldiacylglycerol is a primary ingredient. As examples of monogalactosyldiacylglycerol which the anti-norovirus agent of the invention may contain, (1) monogalactosyldiacylglycerol in which a constituent fatty acid is C16:3 and C18:3, (2) monogalactosyldiacylglycerol in which a constituent fatty acid is C16:2 and C18:3, (3) monogalactosyldiacylglycerol in which a constituent fatty acid is C18:3 and C18:3, (4) monogalactosyldiacylglycerol in which a constituent fatty acid is C16:2 and C18:2, (5) monogalactosyldiacylglycerol in which a constituent fatty acid is C18:3 and C18:2, and (6) monogalactosyldiacylglycerol in which a constituent fatty acid is C16:1 and C18:2 are mentioned. These kinds of monogalactosyldiacylglycerol are identified as those which a microalga extract of the genus Coccomyxa exhibiting anti-norovirus activity contains (the details thereof is referred to "9. Identification 2 of components in MGDG preparation" shown in the section of Examples described below). As also taking the analysis results of "8. Identification 1 of components in MGDG preparation" shown in the section of Examples described below into consideration, the constituent fatty acid C18:3 of the above-described (1) is preferably C18:3(n-3), at least one of the constituent fatty acids C18:3 of the above-described (3) is preferably C18:3(n-3), the constituent fatty acid C18:2 of the above-described (4) is preferably C18:2(n-6), the constituent fatty acid C18:3 of the above-described (5) is preferably C18:3(n-3), and the constituent fatty acid C18:2 of the above-described (5) is preferably C18:2(n-6). Although it is not excluded that a specific monogalactosyldiacylglycerol is contained alone, generally, two or more types of monogalactosyldiacylglycerol having different structures are contained in the anti-norovirus agent of the invention, and the combination, the content ratio, and the like in this case are not particularly limited. The anti-norovirus agent of the invention contains preferably two or more of the above-described (1) to (6), more preferably three or more of the above-described (1) to (6), further more preferably four or more of the above-described (1) to (6), still further preferably five or more of the above-described (1) to (6), and even still further preferably all of the above-described (1) to (6). As shown in Examples described below, particularly high activity was recognized in the monogalactosyldiacylglycerol (MGDG5) of (5). Herein, in an anti-norovirus agent of a particularly preferred embodiment, the monogalactosyldiacylglycerol of (5) is contained alone or is contained in combination with one or more of (1), (2) to (4), and (6).

The monogalactosyldiacylglycerol, which is identified as a main component contained in the microalga extract of the genus Coccomyxa exhibiting anti-norovirus activity, itself can be expected to exhibit anti-norovirus activity. In this regard, according to an embodiment of the invention, there is provided an anti-norovirus agent containing, as an effective ingredient, one or more of monogalactosyldiacylglycerol selected from the group consisting of the above-described (1) to (6). The combination, the content ratio, and the like in the case of containing two or more kinds of monogalactosyldiacylglycerol as effective ingredients are not particularly limited. Also in this embodiment, the anti-norovirus agent contains preferably two or more of the above-described (1) to (6), more preferably three or more of the above-described (1) to (6), further more preferably four or more of the above-described (1) to (6), still further preferably five or more of the above-described (1) to (6), and even still further preferably all of the above-described (1) to (6). As described above, particularly high activity was recognized in the
monogalactosyldiacylglycerol (MGDG5) of (5). Herein, in a particularly preferred embodiment, the
monogalactosyldiacylglycerol of (5) is at least one of effective ingredients.

Herein, there is monogalactosyldiacylglycerol that contains various fatty acids (examples of the fatty acid are described below) as a constituent fatty acid. As taking the teachings of the invention into consideration, monogalactosyldiacylglycerol, including known monogalactosyldiacylglycerol, can be reasonably expected to generally exhibit anti-norovirus activity.

### <Examples of fatty acid>

C12:0, C13:0, C14:0, C14:1, C14:2, C15:0, C15:1, C16:0, C16:1, C16:4, C17:0, C17:1, C18:0, C18:1, C18:4, C19:0, C19:1, C20:0, C20:1, C20:2, C20:3, C20:4, C20:5, C22:0, C22:5, C24:0

### (2) Alga body or dry powder thereof

In this embodiment, an alga body or dry powder thereof is used as an effective ingredient instead of an extract from an alga body. The dry powder can be prepared by subjecting the recovered alga body to a dry treatment and a crushing (pulverizing) treatment. As the dry treatment, for example, drum dry, spray dry, freeze dry, and the like can be employed. In the crushing treatment, a bead type crusher, a homogenizer, a French press, a mixer/blender, a fine pulverizer, and the like can be utilized. The order of the dry treatment and the crushing treatment is not limited. Furthermore, the dry treatment and the crushing treatment may be simultaneously performed by utilizing a device equipped with drying and crushing functions.

The particle diameter of the dry powder is not particularly limited. For example, the average particle diameter of the dry powder is 0.2 µm to 2 mm and preferably 0.4 µm to 400 µm.

### 3. Use application and using method of anti-norovirus agent

The anti-norovirus agent of the invention can be typically used, as a composition containing the anti-norovirus agent, for infection control of norovirus. Examples of the composition described herein include medicines, food, and feed.

As one of specific examples of use application of the anti-norovirus agent of the invention, purification of oysters contaminated by a norovirus can be exemplified (see Examples described below). In this use application, typically, a solution of the anti-norovirus agent is prepared, and oysters contaminated by a norovirus (or oysters that are possibly contaminated) are immersed in the solution (for example, may be reared for approximately several hours to several days).

The medicine of the invention can exhibit a therapeutic effect or a prophylactic effect (these two effects are collectively called "medicinal effects") with respect to norovirus infectious diseases. The medicinal effects described herein include (1) inhibition of norovirus infection, (2) inhibition, suppression, or delay of occurrence of norovirus infectious diseases, (3) relief (alleviation) of symptom characteristic of norovirus infectious diseases or collateral symptom, (4) inhibition, suppression, or delay of ingravescence of symptom characteristic of norovirus infectious diseases or collateral symptom, and the like. Incidentally, the therapeutic effect and the prophylactic effect are partly overlapping concepts, and thus it is difficult and slightly beneficial to clearly discriminate them in some cases.

The formulation of the medicine can be performed according to a common procedure. When formulated, pharmaceutically acceptable other components (for example, a carrier, an excipient, a disintegrating agent, a buffering agent, an emulsifying agent, a suspending agent, a soothing agent, a stabilizer, a preservative, an antiseptic, a normal saline solution, and the like) can be contained. Examples of the excipient that can be used include lactose, starch, sorbitol, D-mannitol, and white sugar. Examples of the disintegrating agent that can be used include starch, carboxymethyl cellulose, and calcium carbonate. Examples of the buffering agent that can be used include phosphates, citrates, and acetates. Examples of the emulsifying agent that can be used include gum arabic, sodium alginate, and tragacanth. Examples of the suspending agent that can be used include glycerol monostearate, aluminum monostearate, methyl cellulose, carboxymethyl cellulose, hydroxymethyl cellulose, and sodium lauryl sulfate. Examples of the soothing agent that can be used include benzyl alcohol, chlorobutanol, and sorbitol. Examples of the stabilizer that can be used include propylene glycol and ascorbic acid. Examples of the preservative that can be used include phenol, benzalkonium chloride, benzyl alcohol, chlorobutanol, and methylparaben. Examples of the antiseptic that can be used include benzalkonium chloride, paraoxybenzoic acid, and chlorobutanol.

The form of formulation is not particularly limited. Examples of the form of formulation include tablets, a powder, fine pellets, granules, capsules, a syrup, an injection, an external preparation (an ointment, a cream, a lotion, a liquid, a gel, a cataplasm, a plaster, a tape, an aerosol, or the like), and suppository. The medicine is administered to the subject orally or parenterally (local injection to the affected part, intravenous, intraarterial, hypodermic, intradermal, intramuscular, or intraperitoneal injection, transdermal, nasotracheal, or transmucosal administration, and the like) in accordance with the formulation. Furthermore, systemic and local administrations are appropriately used according to the subject. These administration routes are not exclusive to each other, and two or more optionally selected may be used in combination.

An amount of the effective ingredient necessary for obtaining an expected effect (that is, a therapeutically or prophylactically effective dose) is contained in the medicine of the invention. The amount of the effective ingredient in the medicine of the invention generally varies depending on the form of formulation, but the amount of the effective ingredient is set, for example, within a range of approximately 0.1 wt% to approximately 99 wt% to achieve the desired dosage amount.

The dosage amount of the medicine of the invention is set so that an expected effect is obtainable. For the setting of the therapeutically or prophylactically effective dosage amount, generally, symptoms, the age, sex, and weight of patients, and the like are taken into consideration. Those skilled in the art can set an appropriate dosage amount in consideration of these factors. As taking the dosage amount as an example, the amount of the effective ingredient for an adult (body weight: approximately 60 kg) per day can be set in such a manner that the dosage amount falls within 1 mg to 20 mg, preferably, 2 mg to 10 mg. The administration schedule can be, for example, once to several times a day, once every two days, once every three days, or the like. For preparation of the administration schedule, the disease state of the patient, the expected duration of the effect of the effective ingredient, and the like can be taken into consideration.

As clearly understood from the above-described description, the present application also provides a method for treating or preventing norovirus infectious diseases, characterized by administering a therapeutically or prophylactically effective dose of a medicine containing the anti-norovirus agent of the invention to a subject affected with norovirus infectious diseases or a subject to be possibly affected with norovirus infectious diseases. The subject for treatment or prevention is typically a human, but the anti-norovirus agent may be applied to mammals (for example, monkey, bovine, pig, sheep, cat, rabbit, dolphin, and the like) other than the human, birds, reptiles, and the like.

As described above, as one of applications of the anti-norovirus agent of the invention, food or feed containing the anti-norovirus agent of the invention is exemplified. Examples of the food of the invention include general foods (grains, vegetables, meat, various processed foods, confectioneries, desserts, milk, refreshing beverages, fruit juice drinks, coffee drinks, extracted juice drinks, alcohol beverages, and the like), dietary supplements (supplements, nutrition drinks, and the like), food additives, foods for pets, and dietary supplements for pets. In the case of dietary supplements or food additives, the form can be a powder, granules, tablets, a paste, a liquid, or the like. The provision in the form of a food composition makes it easy to take the effective ingredient of the invention routinely and continuously.

Examples of the feed of the invention include animal feed (for example, feed for domestic livestock) and pet food.

The food or feed of the invention preferably contains such an amount of the effective ingredient that the therapeutic or prophylactic effect can be expected. The addition amount can be determined in consideration of the state, age, sex, weight, and the like of a subject for which the food or feed is used.

### Examples

### 1. Preparation of dry powder (alga body) and extract of microalga coccomyxa sp. KJ strain and refinement of extract

The coccomyxa sp. KJ strain was cultured according to a previously reported method. Specifically, the coccomyxa sp. KJ strain was seeded to an AF6 culture medium, 2% CO₂ (v/v) was then ventilated, and culturing was performed at room temperature (25°C) for 48 hours with irradiation of light (300 µmol/m²/s). An alga body was recovered from a culture solution by centrifugal separation. The recovered alga body was dried by a drum drier and crushed by a fine pulverizer to be formed into a powdery form (dry powder of the alga body). Next, one liter of ethanol was added to 100 g of the dry powder and dispersed, and the resultant product was left to stand still in the dark place for 3 days. After being left to stand still, the resultant product was filtered to be separated into a primary filtrate and the residue. To this residue, one liter of ethanol was added similarly to the above description and dispersed, and the resultant product was left to stand still for 3 days and filtered again to be separated into a secondary filtrate and the residue. This filtration operation was repeated once more to obtain a tertiary filtrate and the residue.

The primary filtrate, the secondary filtrate, and the tertiary filtrate were mixed, ethanol was distilled by an evaporator, and drying under reduced pressure was performed, thereby obtaining an ethanol extract DE. Next, the DE was subjected to DIAION HP-20 (manufactured by Mitsubishi Chemical Corporation, 3.5 x 57 cm) column chromatography. The elutions were performed with H₂O, 50% ethanol (EtOH), and EtOH and acetone, consecutively, and each eluted fraction was dried under reduced pressure at room temperature (DE1, 400.9 mg; DE2, 310.3 mg; DE3, 2.25 g; DE4, 4.86 g). The acetone fraction (DE4) was subjected to silica gel column (3 x 42 cm) chromatography. The elutions were performed with hexane, hexane-ethyl acetate (AcOEt) (1 : 1), AcOHt, AcOHt-acetone (1 : 1), and acetone and methanol (MeOH), consecutively, and each eluted fraction, that is, DE4A (55.4 mg), DE4B (2.0 g), DE4C (89.0 mg), DE4D (1.01 g), DE4E (95.5 mg), and DE4F (177.1 mg) were obtained. Next, DE4D was subjected to silica gel column (1.5 x 35 cm) chromatography using AcOEt-acetone as a solvent system, and three fractions, that is, DE4D1 (49.9 mg), DE4D2 (705.1 mg), and DE4D3 (16.2 mg) were obtained. Subsequently, DE4D2 was subjected to silica gel column (1.5 x 35 cm) chromatography and eluted with CHCl₃-MeOH-H₂O (10 : 1 : 0.1), thereby obtaining DE4D2A (15.6 mg) and DE4D2B (686.5 mg). Among these, DE4D2B was subjected to LH-20 column (manufactured by Sigma-Aldrich Co. LLC) chromatography using chloroform (CHCl₃)-MeOH (3 : 1) as a solvent system, and DE4D2Bl (11.6 mg) and DE4D2B2 (652.3 mg) were obtained. DE4D2B2 was subjected to silica gel column (2 x 50 cm) chromatography and eluted with CHCl₃, CHCl₃-MeOH (20 : 1), and CHCl₃-MeOH-H₂O (10 : 3 : 1), thereby obtaining four fractions, that is, DE4D2B2A (4.8 mg), DE4D2B2B (3.5 mg), DE4D2B2C (25.1 mg), and DE4D2B2D (620.5 mg). Finally, DE4D2B2D was refined by preparative liquid chromatography (PLC) developed with CHCl₃-MeOH-acetate (AcOH)-H₂O (80 : 9 : 12 : 2) to obtain an MGDG preparation (578.9 mg).

### 2. Murine norovirus (MNV) infection experiment 1 (effect of alga body of coccomyxa sp. KJ strain)

Human noroviruses become a main cause of infectious gastroenteritis. Until now, artificial culturing of these viruses has not achieved success, and this becomes a barrier of development of vaccines or therapeutic agents. Therefore, murine noroviruses (MNV) are used as an alternate pathogen in searching for infectious disease measures in many cases. Herein, the infectious disease treatment effect of the alga body (dry powder) of the coccomyxa sp. KJ strain with respect to the norovirus infectious diseases was evaluated by using the MNV infection system. It is known that the gastroenteritis symptom becomes severe at the time of diminishing the immune function, and thus an influence of a difference between immune functions on virus excretion or antibody production was also examined.

### <Method>

(1) BALB/c mice (6-week old) are divided into A and B based on each administration group, A (normal immune function group, n = 3) is not administered with 5-fluorouracil (5-FU), and B (diminished immune function group, n = 3) is subcutaneously injected with 5-FU (0.25 mg/day/mouse) from 7 days before virus inoculation to 21 days after virus inoculation every other day.
(2) MNV (1 x 10⁶ PFU/0.2 ml/mouse) is orally inoculated.
(3) The sample (the dry powder of the alga body) is orally administered at the following dosage amount from 7 days before virus inoculation to 21 days after virus inoculation (twice a day, at 9 o'clock and 18 o'clock).
   Control 1A: administration of sterilized water; 5-FU untreated
   Control 1B: administration of sterilized water; 5-FU treated
   Test section 2A: Alga body (dry powder) 5 mg/0.4 ml/day; 5-FU untreated
   Test section 2B: Alga body (dry powder) 5 mg/0.4 ml/day; 5-FU treated
(4) The feces of each mouse is collected by a 5 ml tube after 8 hours, 1 day, 2 days, 3 days, 4 days, 5 days, 6 days, 8 days, 10 days, 12 days, 14 days, 16 days, 18 days, and 21 days, and is stored at -80°C (mice are put in an empty rearing cage for 15 to 30 minutes).
(5) 10 µl of PBS per 1 mg of the feces is added and subjected to an ultrasonic treatment to be uniformly dispersed.
(6) A centrifugal treatment is performed (3,000 rpm, 15 min, 4°C).
(7) The supernatant is diluted with PBS (10⁰-fold, 10¹-fold, 10²-fold, and 10³-fold).
(8) 100 µl/well of each diluted solution of (7) is added to RAW 264.7 cells, which have been cultured in a 24-well plate a day before, after removing the culture medium, and the virus is inoculated to the cells at room temperature for 1 hour.
(9) After removing the virus fluid, a 1.5% SeaPlaque™ agarose-added DMEM culture medium is overlaid.
(10) After 2 days, a neutral red solution is overlaid at 500 µl/well and treated at 37°C for 1 hour.
(11) After removing the neutral red solution, the number of plaques is immediately measured under a microscope.
(12) The whole blood is collected 21 days after infection, a centrifugal treatment is performed (3,000 rpm, 5 min, 4°C) to separate the blood serum, and then the neutralizing antibody titer is measured by the following method.
   (i) The blood serum is diluted with PBS (10-fold, 50-fold, 200-fold, 1000-fold, and 5000-fold).
   (ii) 100 µl of the virus (2000 PFU/ml) is mixed with 100 µl of the above-described diluted solution (200 PFU/200 µl/well) (PBS is added to the control instead of the blood serum).
   (iii) The treatment is performed at 37°C for 1 hour.
   (iv) The above-described mixed liquid is added to RAW 264.7 cells, which have been cultured in a 24-well plate in a monolayer fashion, in 100 µl (= approximately 100 PFU) per 1 well, and the virus is inoculated to the cells at room temperature for 1 hour.
   (v) A 1.5 % SeaPlaque™ agarose-added DMEM (not containing FBS) is overlaid at 500 µl/well.
   (vi) After 2 days to 3 days, approximately 500 µl/well of a neutral red solution is added and treated at 37°C.
   (vii) The neutral red solution is removed after 1 hour, the number of plaques is counted under a microscope.
   (viii) The relative plaque number (%) of each diluted solution is calculated based on the number of plaques of the control (100%). The dilution factor of the blood serum inhibiting 50% of plaque formation is determined on the graph, and this is regarded as the neutralizing antibody titer.

### <Result and Discussion>

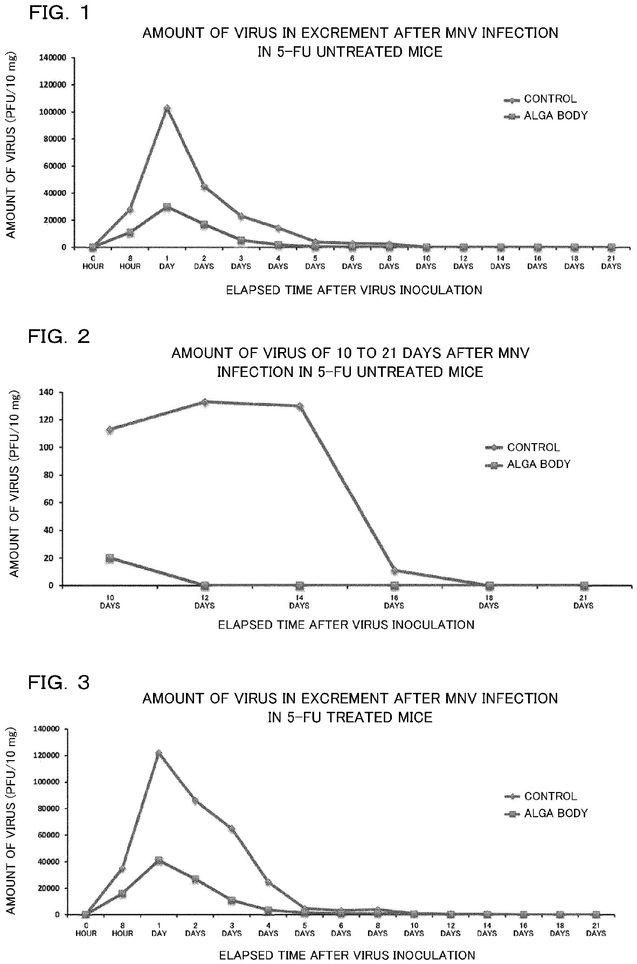

### (1) Amount of virus in feces (Figs. 1 to 5)

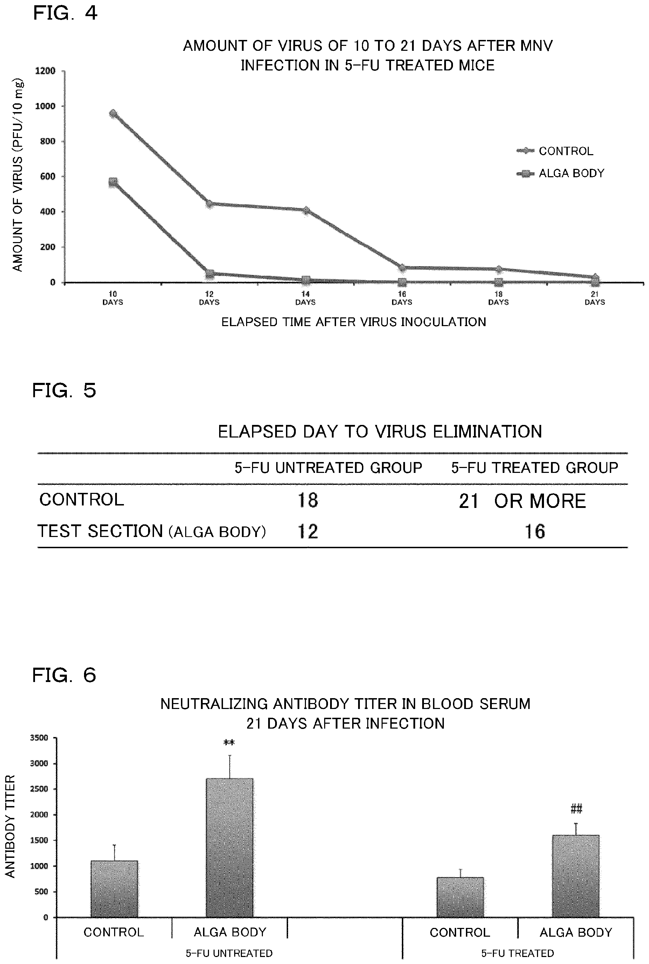

The excretion amount of virus was maximized 1 day after the infection (Figs. 1 and 3). In the 5-FU treated group, as compared to the untreated group, the excretion amount of virus was large, and the virus was excreted for a long period of time (Figs. 1 to 4). In particular, in the control group, the virus was still detected 3 weeks after the infection (Fig. 4). The alga body of the coccomyxa sp. KJ strain suppressed virus production in both the 5-FU untreated group and the 5-FU treated group, and stopped virus excretion in an early stage as compared to the control group (Fig. 5).

### (2) Neutralizing antibody titer (Fig. 6)

In the 5-FU untreated group, by administration of the alga body of the coccomyxa sp. KJ strain, the antibody titer was significantly increased (p < 0.01). At the time of the 5-FU treatment, the antibody titers in both groups were lower values than that of the corresponding 5-FU untreated group, but by administration of the alga body of the coccomyxa sp. KJ strain, a significant (p < 0.01) increase in antibody titer was shown, and this antibody titer was a higher value than that of the 5-FU untreated control group.

The above results can be discussed as follows.
- In the case of diminishing the immune function, excretion of the norovirus from the intestinal canal persists for a long time, and it is speculated that this is involved with a low neutralizing antibody production amount.
- In the alga body of the coccomyxa sp. KJ strain, at the time of both the normal immune function and the diminished immune function, effects of decreasing the amount of norovirus in the intestinal canal and shortening the period of excretion of the virus were recognized. It is considered that an increase in neutralizing antibody amount contributes to this.

### 3. Murine norovirus (MNV) infection experiment 2 (effect of MGDG preparation of coccomyxa sp. KJ strain)

### <Method>

The experiment was performed by the same method as in the experiment of the above 2. A sample was an MGDG preparation and was orally administered at a dosage of 1 mg/day twice a day.
Control 1A: administration of sterilized water; 5-FU untreated
Control 1B: administration of sterilized water; 5-FU treated
Test section 2A: MGDG preparation 1 mg/0.4 ml/day; 5-FU untreated
Test section 2B: MGDG preparation 1 mg/0.4 ml/day; 5-FU treated

### <Result and Discussion>

During observation for 21 days, slightly soft feces was observed in some of mice treated by 5-FU injection, but a decrease in body weight was not recognized, and all the mice survived.

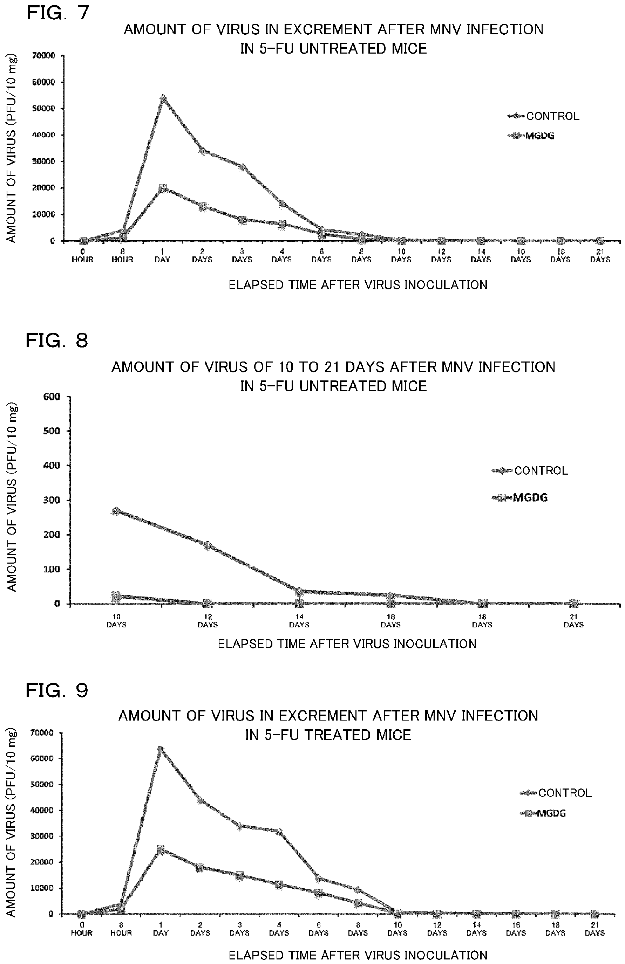

### (1) Amount of virus in feces (Figs. 7 to 11)

In the case of normal immune function mice (5-FU untreated) (Figs. 7, 8, and 11), in the control group, the amount of virus was maximized 1 day after the infection and then was gradually decreased, and the virus was not detected 18 days after. On the other hand, in the MGDG preparation administration group, the amount of virus of 1 day after the infection was decreased to 1/3 of the amount of virus in the control group, and even thereafter, the amount of virus was consistently a low value. Further, in the MGDG preparation administration group, the virus in the feces was eliminated 14 days after the infection.

In the case of the diminished immune function mice (5-FU treated) (Figs. 9, 10, and 11), the excretion amount of the virus was increased by the 5-FU treatment, and the period of egestion was also lengthened. In particular, in the control group, the virus was still detected 21 days after. On the other hand, the amount of virus was decreased over the 21 days by administration of the MGDG preparation. In the MGDG preparation administration group, the virus was not detected 14 days after.

As described above, virus proliferation in the intestinal canal is suppressed by administration of the MGDG preparation, and virus excretion is stopped in an early stage.

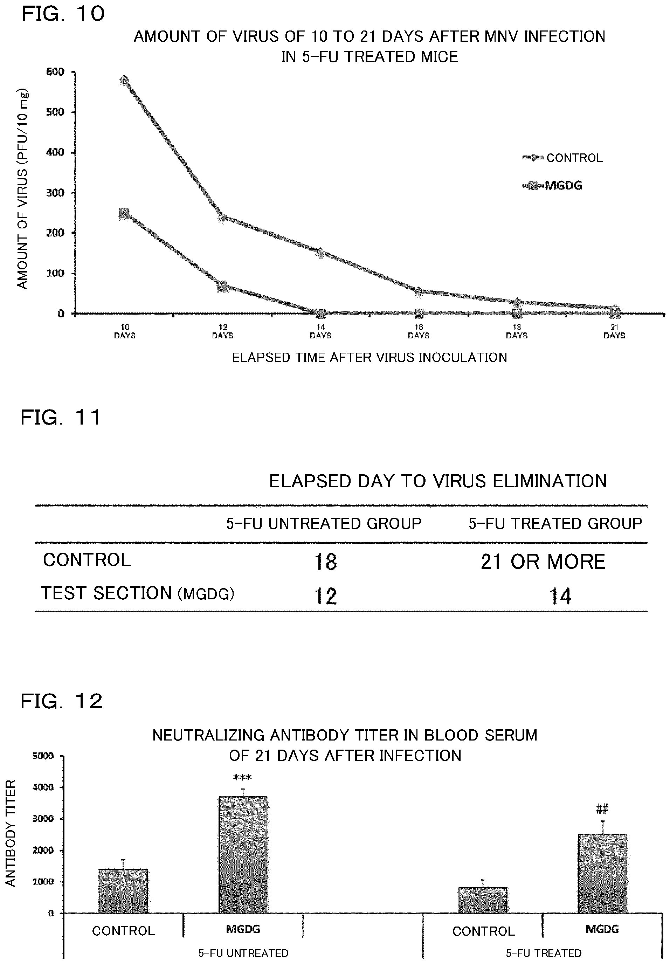

### (2) Neutralizing antibody titer (Fig. 12)

After three weeks of infection, the amount of a neutralizing antibody (an antibody eliminating infectability of MNV) was measured. In the case of normal immune function mice (5-FU untreated), in the MGDG preparation administration group, the antibody titer was significantly increased (p < 0.001) as compared to the control group. Furthermore, in the case of diminished immune function mice (5-FU treated), as compared to the 5-FU untreated group, the neutralizing antibody titers in both the control group and the MGDG preparation administration group were low. By administration of the MGDG preparation administration, a significantly (p < 0.01) higher antibody titer than that of the control group was obtained, and this antibody titer was higher than the antibody titer of the 5-FU untreated control group.

From the above results, it is speculated that an increase in amount of the antibody by administration of the MGDG preparation causes norovirus proliferation in the intestinal canal to be suppressed. Furthermore, even in a state where the immune function is diminished, the MGDG preparation stops virus egestion in an early stage, and thus the MGDG preparation can be expected to have an effect of inhibiting infection spread to the surroundings from an infected patient.

### 4. Virucidal activity targeting feline calicivirus

Also regarding feline calicivirus (FCV) used as a replacement for norovirus, evaluation on whether or not the MGDG preparation exhibits virucidal activity was conducted.

### <Method>

(1) The stock of the virus (FCV) is diluted with phosphate buffered saline (PBS) to prepare 2×10⁵ PFU/ml of a virus fluid.
(2) 0.5 ml of a sample (MGDG preparation having a predetermined concentration) and 0.5 ml of the virus fluid are put into a 1.5 ml microtube and then mixed. Incidentally, in the control, PBS is added instead of the sample.
(3) The mixed liquid of the sample and the virus fluid is placed at room temperature, and 10 µl of the mixed liquid is collected at each lapse time after 0 minute, 1 minute, 10 minutes, 30 minutes, and 60 minutes and mixed with 990 µl of PBS (100-fold dilution). Incidentally, this dilution is performed to prevent the influence of the sample at the time of measuring the amount of the survival virus which will be performed below.
(4) 100 µl of a 100-fold diluted solution is added to host cells which have been cultured in a monolayer fashion in advance (feline kidney-derived CRFK cells) and the virus is inoculated to the cells at room temperature for 1 hour.
(5) A culture medium for plaque assay is overlaid and culturing is performed at 37°C for 2 to 3 days.
(6) After checking appearance of plaques, the cells are immobilized and stained with a neutral red solution, and the number of plaques is measured under a microscope.
(7) The amount of the survival virus (relative plaque number after each treatment time is calculated based on the number of plaques at 0 hour (100%).

### <Result and Discussion>

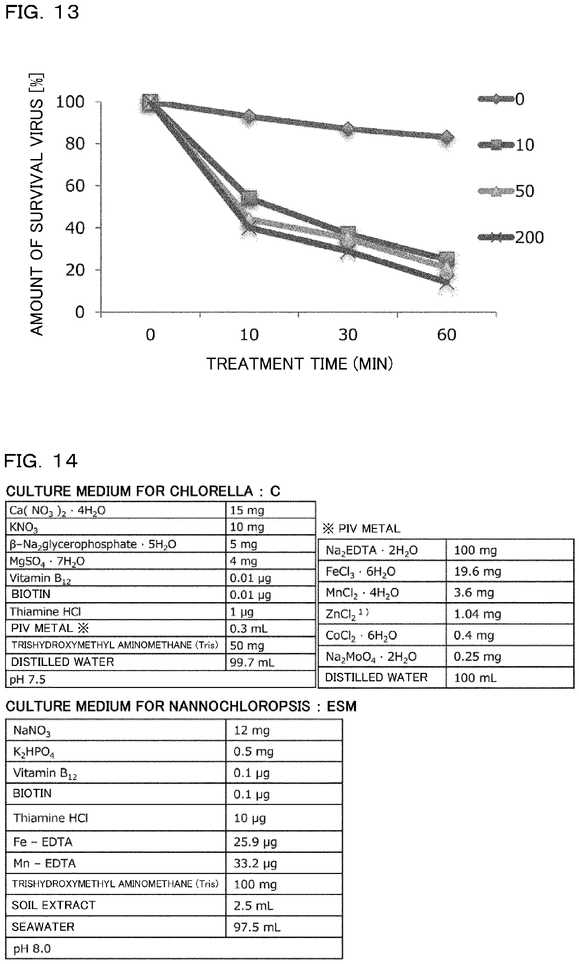

The MGDG preparation (10 to 200 µg/ml) exhibits virucidal (inactivation) activity concentration-dependently or time-dependently with respect to FCV (Fig. 13).

### 5. Preparation of MGDG of Chlorella vulgaris, Nannochloropsis oculata, Arthrospira platensis (spirulina), and Euglena gracilis

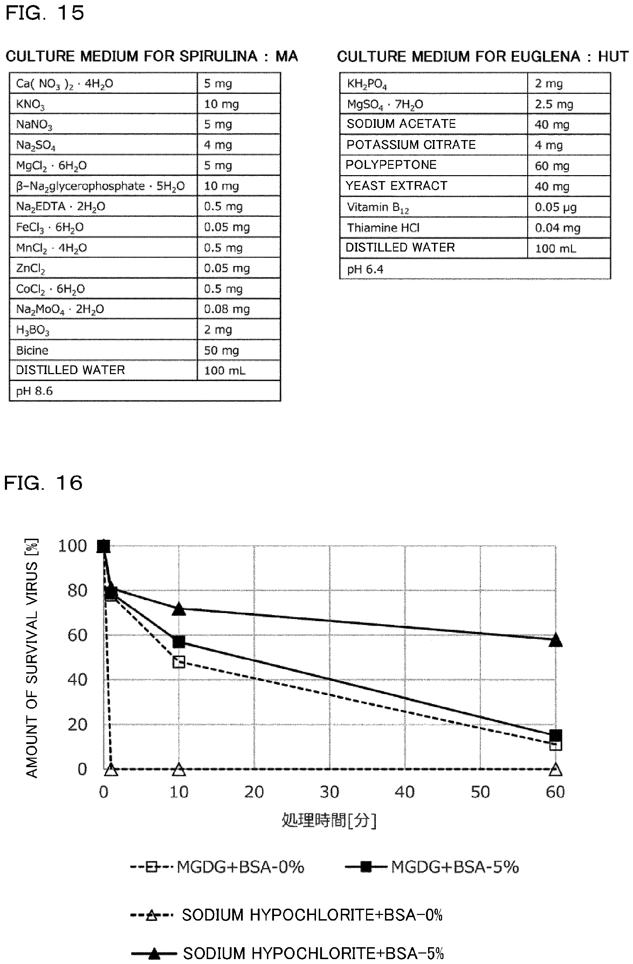

MGDG is extracted from Chlorella vulgaris, Nannochloropsis oculata, Arthrospira platensis (spirulina), and Euglena gracilis. The culturing of each alga may be performed according to an ordinary method by using a culture medium suitable for each alga (for example, in the case of Chlorella vulgaris, a C culture medium shown in Fig. 14, in the case of Nannochloropsis oculata, an ESM culture medium shown in Fig. 14, in the case of Arthrospira platensis (spirulina), an MA culture medium shown in Fig. 15, and in the case of Euglena gracilis, an HUT culture medium shown in Fig. 15) (the same culture conditions as in the above-described coccomyxa sp. KJ strain may be employed). The alga body recovered from the culture solution by centrifugal separation is dried by a drum drier and crushed by a fine pulverizer, and thereby dry powder of each alga body can be obtained. An MGDG preparation of each alga can be obtained from the dry powder by the same extraction operation as in the case of the coccomyxa sp. KJ strain. However, in studies this time, processed products of respective algae ("Chlorella vulgaris dry powder" (AOI TEA COMPANY) for Chlorella vulgaris, "Nannochloropsis oculata frozen product" (Marinetech) for Nannochloropsis oculata, "Spirulina powder" (DIC LIFETEC Co., Ltd.) for Arthrospira platensis (spirulina), "Inochino Euglena Kiwami" (SixthsenseLab Corporation) for Euglena gracilis, and Biozyme (Euglena) were purchased and subjected to extraction of MGDG (the extraction operation is the same as in the case of the coccomyxa sp. KJ strain). Each MGDG preparation thus obtained is used for the following studies. An MGDG preparation was obtained also from commercially available spinach (Spinacia oleracea) by performing crushing with a fine pulverizer after freeze dry and extracting MGDG.

### 6. Virucidal activity targeting norovirus condensed in oysters (purification test of oysters contaminated by norovirus)

### (1) Production of contaminated oysters

Live oysters are purchased, the surfaces of the shells are thoroughly washed under running tap water by using a brush, and then the oysters are left to stand still in a clean bench so that the shell surfaces are sterilized under ultraviolet rays. Next, the oysters having the shell surfaces treated with ultraviolet rays are put into seawater (water temperature: 10°C) filtered (pore diameter: 0.2 µm; manufactured by Millipore Corp.) and sterilized, and the oysters are reared in the fasting state for twenty-four hours under ventilation. Next, the stock of murine noroviruses having been prepared in advance is put into this filtered and sterilized seawater so that the final concentration becomes 2×10⁵ PFU/ml. Then, the oysters are further reared for twenty-four hours, and oysters contaminated by murine noroviruses are produced.

### (2) Production of MGDG preparing seawater

DMSO is added to the MGDG preparations (the coccomyxa sp. KJ strain MGDG preparation, the Chlorella vulgaris MGDG preparation, the Nannochloropsis oculata MGDG preparation, the Arthrospira platensis (spirulina) MGDG preparation, the Euglena gracilis MGDG preparation, and the Spinacia oleracea MGDG preparation) or MGDG obtained by refining these, and uniformly suspended (MGDG suspension). Next, the MGDG suspension is added to 10 L of filtered (pore diameter: 0.2 µm; manufactured by Millipore Corp.) and sterilized seawater so that the final concentration of MGDG becomes 100 ppm, and thereby cloudy MGDG preparing sterilized seawater is obtained.

### (3) Purification test

In an MGDG treatment section, contaminated oysters are stored one by one in a beaker with 1 L of filtered and sterilized seawater of 100 ppm of various kinds of MGDG. In the control section (without MGDG treatment), contaminated oysters are stored one by one in a beaker with 1 L of filtered and sterilized seawater without addition of MGDG for comparison. The seawater temperature is set to 10°C and the oysters are reared for 48 hours under ventilation. After a lapse of 48 hours, the oysters are taken out from the seawater, the oyster meats are taken out from the shells and placed on filter paper to thoroughly wipe off moisture. Then, the oyster meats are put in a plastic bag and cryogenically stored at -80°C. By this operation, the murine noroviruses accumulated in the oysters are maintained without flowing out. Incidentally, the number of times of the test is set to a sufficient number for obtaining a significant difference by comparison.

### (4) Evaluation of survival murine noroviruses

Since the murine noroviruses do not proliferate in the oyster digestive canal, basically, it is considered that all of the added murine noroviruses are incorporated into the oysters and accumulated in the digestive canal. The oysters frozen in (3) are defrosted, the digestive canal is excised from the oysters, the inside of this digestive canal is washed with a certain amount of phosphate buffered saline (PBS), and the washer fluid is diluted with PBS 100-fold. 100 µl of this 100-fold diluted solution is added to host cells which have been cultured in a monolayer fashion in advance (RAW264.7 cells that are murine macrophage-like cells) and the virus is inoculated to the cells at room temperature for 1 hour. Then, a culture medium for plaque assay is overlaid and culturing is performed at 37°C for 2 to 3 days. After checking appearance of plaques, the cells are immobilized and stained with a neutral red solution, and the number of plaques is measured under a microscope. The amount of the viable virus in the oyster digestive canal after 48 hours (relative plaque number) is calculated based on the initial concentration of the murine noroviruses of 2×10⁵ PFU/ml (100%).

In the MGDG treatment section, the murine noroviruses that have contaminated the oysters lose the infection performance by contact with the incorporated MGDG, and the result that plaques rarely occur from the host cells is obtained.

### 7. Virucidal effect in presence of organic material

Noroviruses are contained in a vomited material or feces of a person infected with noroviruses, and secondary infection due to these becomes a problem. Furthermore, the virucidal effect for noroviruses is considered to be low in an alcoholic disinfectant that is generally used, and thus a chlorine-based disinfectant is mainly used. However, chlorine is decomposed by an organic material contained in a vomited material, feces, or the like, and thus the virucidal effect is attenuated in the chlorine-based disinfectant. In this regard, whether the MGDG preparation exhibits the virucidal effect even in the presence of the organic material was studied.

### <Used virus, reagent, etc.>

Virus: using feline calicivirus (FCV) as a replacement for norovirus
Used medicine and concentration: 1,000 µg/ml of sodium hypochlorite or coccomyxa sp. KJ strain MGDG preparation
Organic material: using BSA (albumin) 5%

### <Method>

(1) Feline calicivirus (FCV) was prepared in 2×10⁵ PFU/ml.
(2) An MGDG preparation or sodium hypochlorite is prepared at a concentration four times the final assay concentration (1,000 pg/ml).
(3) Bovine serum albumin (BSA) is prepared as an organic material at a concentration four times the final concentration (5%).
(4) Virus fluid: the MGDG preparation or sodium hypochlorite and BSA are mixed at a ratio of 2 : 1 : 1. A sample prepared by adding PBS instead of the MGDG preparation or sodium hypochlorite was used as a control.
(5) After being left at room temperature for 1 minute, 10 minutes, and 60 minutes, 100-fold dilution with PBS is performed. The CRFK cells cultured in a monolayer fashion are added in an amount of 100 µl/culture dish to a 35 mm culture dish, and an infection treatment is performed at room temperature for 1 hour.
(6) A 1% SeaPlaque Agarose-added MEM culture medium is overlaid.
(7) After 2 days, the appeared plaque is immobilized and stained with a crystal violet solution.
(8) The number of plaques is counted under a microscope.

### <Result>

As shown in Fig. 16, in a case where the organic material (BSA) did not exist, sodium hypochlorite inactivated the virus in one minute at a rate of 100%. However, the inactivation effect was significantly reduced in the presence of 5% of the organic material. On the other hand, the inactivation effect of MGDG was almost not changed even in the presence of the organic material, and a high inactivation effect was exhibited as compared to sodium hypochlorite.

### 8. Identification 1 of components in MGDG preparation

By HPLC analysis, it was estimated that five types of MGDG (called MGDG1, MGDG2, MGDG3, MGDG4, and MGDG5) are contained in the coccomyxa sp. KJ strain MGDG preparation. In order to clearly determine the structures of these MGDG, the fatty acid composition was analyzed by using gas chromatography (GC/FID). In the analysis, BPX90 column (length 100 m × inner diameter 0.25 mm, film thickness 0.25 µm) was used and hydrogen was used as a carrier gas. FAME Standard (F.A.M.E. Mix, C4-C24, manufactured by SUPELCO) and MGDG (MGDG plant, manufactured by Avanti) were used as a standard sample for identification.

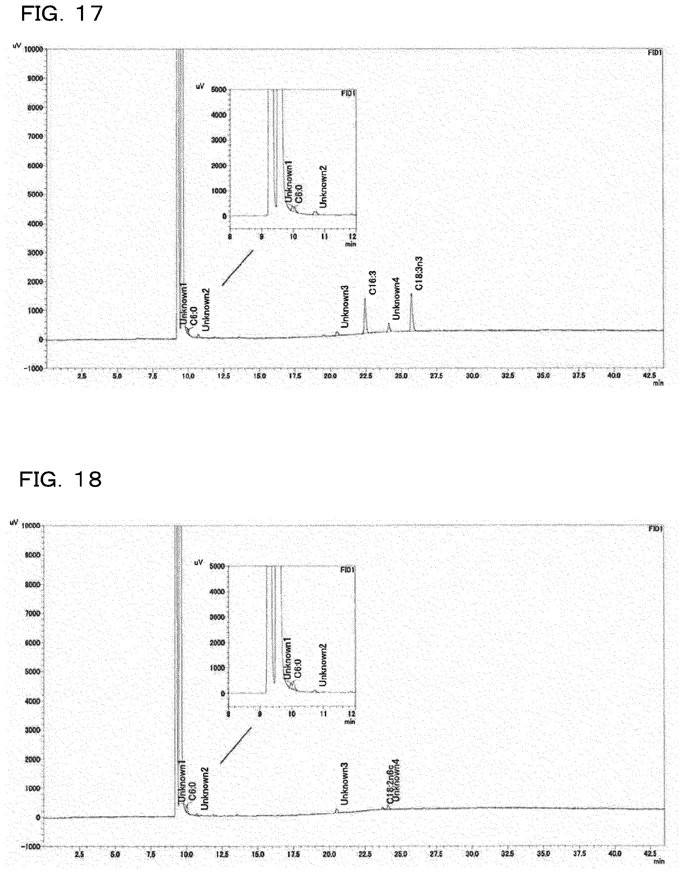

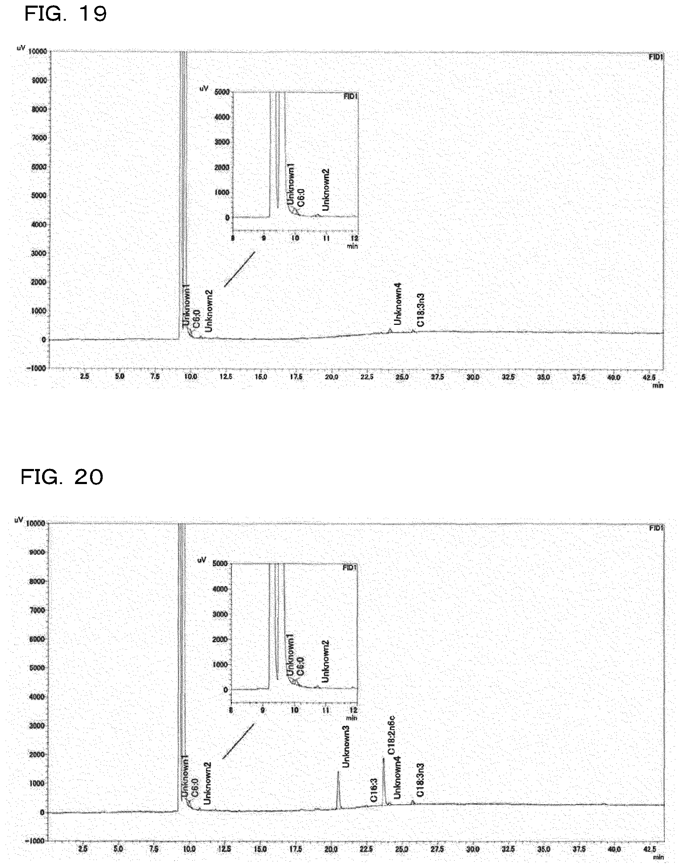

The chromatogram of each sample (MGDG1, MGDG2, MGDG3, MGDG4, MGDG5) is shown in Figs. 17 to 21. Herein, as for C6:0, it is general in a fatty acid composition of general lipid that fatty acid having a close number of carbon atoms (even number) is also contained in combination; however, this was almost not detected by analysis this time, and thus it is considered that the possibility that this peak is likely not to be fatty acid is high. Furthermore, Unknown 1 and Unknown 2 are considered to have a high possibility that they are components other than fatty acid since it is difficult to assume fatty acid to be detected in the retention time of these peaks in general lipid. On the other hand, from the retention time, Unknown 3 and Unknown 4 are estimated to be C16:2 and C18:2 (other than n-6), respectively.

The analysis results are shown in the following Table 1 and Table 2. Table 1 shows the fatty acid composition ratio of each sample by an area percentage method. Table 2 collectively shows results obtained by calculation excluding C6:0 and unknown components 1 and 2 (Unknown 1 and Unknown 2). Incidentally, as for MGDG3, the area ratio of two components is 3 : 1, which is extremely biased. This can be interpreted that, in the combination of fatty acid residues, two kinds of MGDG, which are (C18:2 (other than n-6) and C18:2 (other than n-6)) and (C18:2 (other than n-6) and C18:3 n3), are mixed at a ratio of approximately 1 : 1.

**[Table 1]**

| Name of Component | MGDG 1 (%) | MGDG 2 (%) | MGDG 3 (%) | MGDG 4 (%) | MGDG 5 (%) |
|---|---|---|---|---|---|
| Unknown 1 | 3.1 | 14.3 | 28.9 | 2.7 | 1.9 |
| C6:0 | 2.6 | 26.1 | 12.6 | 3.2 | 1.3 |
| Unknown 2 | 2.8 | 9.1 | 13.8 | 1.4 | 1.4 |
| C16:2(Estimated) | 5.2 | 23.2 | - | 38.9 | 1.7 |
| C16:3 | 36.5 | - | - | 1.1 | 1.0 |
| C18:2n6c | - | 6.3 | - | 47.0 | 46.8 |
| C18:2(other than n-6) (Estimated) | 9.0 | 21.0 | 32.6 | 1.9 | 2.8 |
| C18:3n3 | 40.8 | - | 12.0 | 3.8 | 43.0 |

| | | | | | |
|---|---|---|---|---|---|
| "-" indicates "not detectable". | | | | | |

**[Table 2]**

| Name of Component | MGDG 1 (%) | MGDG 2 (%) | MGDG 3 (%) | MGDG 4 (%) | MGDG 5 (%) |
|---|---|---|---|---|---|
| C16:2(Estimated) | 5.6 | 45.9 | - | 42.0 | 1.8 |
| C16:3 | 39.9 | - | - | 1.2 | 1.1 |
| C18:2n6c | - | 12.5 | - | 50.7 | 49.1 |
| C18:2(other than n-6)(Estimated) | 9.8 | 41.6 | 73.1 | 2.1 | 3.0 |
| C18:3n3 | 44.6 | - | 26.9 | 4.1 | 45.1 |

| | | | | | |
|---|---|---|---|---|---|
| "-" indicates "not detectable". | | | | | |

As described above, it became clear that (1) monogalactosyldiacylglycerol (MGDG1) in which a constituent fatty acid is C16:3 and C18:3(n-3), (2)
monogalactosyldiacylglycerol (MGDG2) in which a constituent fatty acid is C16:2 and C18:2, (3)
monogalactosyldiacylglycerol (MGDG3) in which a constituent fatty acid is C18:2 and C18:3(n-3), (4)
monogalactosyldiacylglycerol (MGDG3) in which a constituent fatty acid is C18:2 and C18:2, (5)
monogalactosyldiacylglycerol (MGDG4) in which a constituent fatty acid is C16:2 and C18:2(n-6), and (6)
monogalactosyldiacylglycerol (MGDG5) in which a constituent fatty acid is C18:2(n-6) and C18:3(n-3) are contained in the MGDG preparation.

### 9. Identification 2 of components in MGDG preparation

The structure of MGDG contained in the coccomyxa sp. KJ strain MGDG preparation was examined in more detail by using a triple quadrupole LC/MS system.

### <Analysis conditions>

Device name: Triple quadrupole LC/MS system
LC column: YMC Triart C-18 (length 100 mm × inner diameter 2.1 mm, particle diameter 1.9 µm)
Mobile phase: methanol, acetonitrile, water, and ammonium acetate
Ion source: ESI
Measurement mode: MS2 scan, Product ion scan

### <Result>

In addition to five types of MGDG detected by preparative liquid chromatography (PLC) (see the section of 8. Identification 1 of components in MGDG preparation), the sixth MGDG was newly detected by triple quadrupole LC/MS analysis. As for these MGDGs (six peaks), from the results of MS scan and Product ion scan, two fatty acid side chains constituting each MGDG (peak 1: MGDG1, peak 2: MGDG2, peak 3: MGDG3, peak 4: MGDG4, peak 5: MGDG5, and peak 6: MGDG6) could be identified. The fatty acid chain of each MGDG is shown in the following table.

**[Table 3]**

| Fatty acid side chain of each MGDG | | |
|---|---|---|
| | Fatty acid side chain | |
| Peak 1 | 16:3 | 18:3 |
| Peak 2 | 16:2 | 18:3 |
| Peak 3 | 18:3 | 18:3 |
| Peak 4 | 16:2 | 18:2 |
| Peak 5 | 18:3 | 18:2 |
| Peak 6 | 16:1 | 18:2 |

### 10. Virucidal activity of each MGDG fraction

The virucidal activity of each MGDG (MGDG1 to MGDG5) constituting the MGDG preparation was compared.

### <Method>

(1) A coccomyxa sp. KJ strain-derived MGDG preparation was subjected to HPLC, and each peak was separated, thereby obtaining five types of MGDG refined products (MGDG1, MGDG2, MGDG3, MGDG4, and MGDG5). Furthermore, the MGDG preparation before separation (containing MGDG1 to MGDG5) was prepared as a mix product.
(2) Each sample was mixed with respect to each virus fluid (2×10⁵ PFU/ml) so that the final concentration became 50 µg/ml, and the resultant product was left to stand still at 37°C for 60 minutes. The used viruses and host cells are shown in Table 4.

**[Table 4]**

| Virus for evaluation and host cell | |
|---|---|
| Virus for evaluation | Host cell |
| Influenza A virus (IFV) | MDCK cell |
| Herpes simplex virus type 2 (HSV-2) | Vero cell |
| Feline calicivirus (FCV) | CRFK cell |
| Poliovirus 1 (Pol-1) | Vero cell |

(3) The mixed liquid of (2) is diluted in the MEM culture medium without foetal bovine serum, a culture medium for plaque assay is then overlaid, and culturing is performed at 37°C for 2 to 3 days.
(4) After checking appearance of plaques, the cells were immobilized and stained with a neutral red solution, and the number of plaques was measured under a microscope. The virus survival rate of each sample was calculated based on the appearance number of virus appearing in the case of not adding the sample (100%). The virucidal performance of each sample was evaluated from the virus survival rate.

### <Result and Discussion>

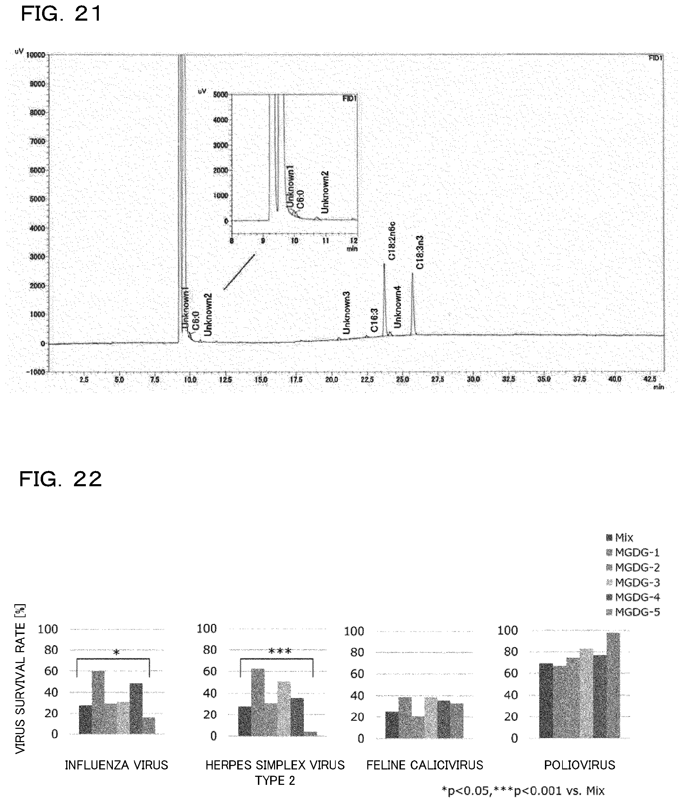

Although there is a difference in activity depending on a target virus, the virucidal (inactivation) activity was recognized in all of the MGDG preparation (Mix product) and MGDG1 to MGDG5 (Fig. 22). MGDG5 exhibited a significant inactivation effect with respect to influenza virus and herpes simplex virus type 2 as compared to the MGDG preparation (Mix product). In the non-enveloped feline calicivirus and poliovirus, there was no sample showing a significantly higher activity than other samples. The above results support the fact that the MGDG preparation and each MGDG constituting this have virucidal activity and indicate the fact that MGDG5 exhibits particularly high virucidal activity with respect to enveloped viruses such as influenza virus and herpes simplex virus type 2.

### 11. Comparison of component in various MGDG preparations

The coccomyxa sp. KJ strain MGDG preparation, the Chlorella vulgaris MGDG preparation, the Nannochloropsis oculata MGDG preparation, the Arthrospira platensis (spirulina) MGDG preparation, the Euglena gracilis MGDG preparation, and the Spinacia oleracea MGDG preparation were analyzed by reverse-phase HPLC, and components contained therein were compared.

### <HPLC analysis conditions>

Device name: Alliance 2695
Column: YMC-Actus Triart C18
Mobile phase: Mixed solution of methanol, water, and acetonitrile
Detection wavelength: 205 nm

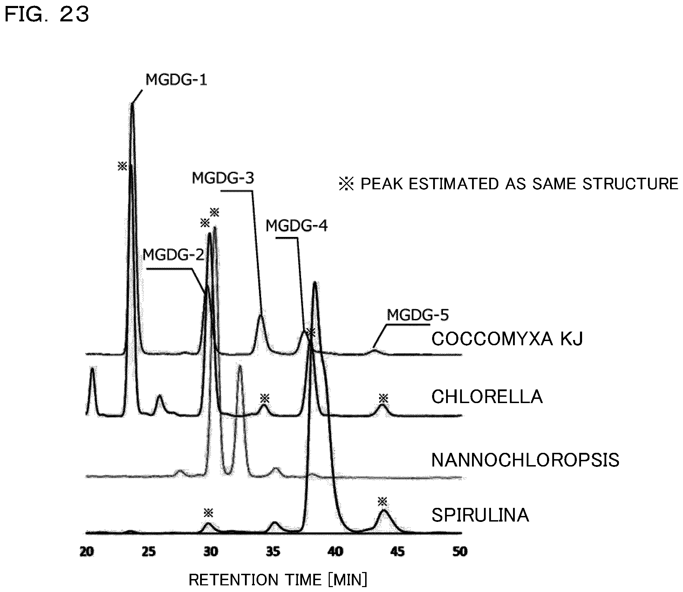

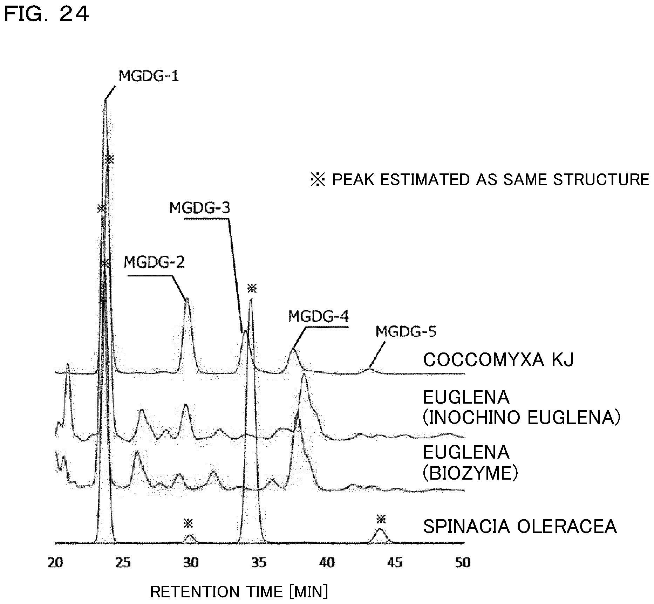

The measurement results (chromatogram) of HPLC are shown in Fig. 23 and Fig. 24. The vertical axis of the chromatogram is standardized such that the peak maximum value in each sample becomes 1.
- In the MGDG preparation of the KJ strain, at least five peaks were detected. On the other hand, also in the MGDG preparation of each organism other than the KJ strain, the retention time is coincident with that of the MGDG preparation of the KJ strain or at least one very close peak is detected. As for peaks in which the retention time is coincident, it is estimated to have the same chemical structure as that of the KJ strain-derived MGDG.
- In five peaks derived from the KJ strain MGDG preparation, the virucidal activity with respect to HSV-2, IFV, and FCV is recognized (Test in the section of 10., Fig. 22). As described above, in the MGDG preparation of each organism other than the KJ strain, at least one peak corresponding to the five peaks derived from the KJ strain MGDG preparation is recognized, and thus it is speculated to similarly have the virucidal activity. That is, the same effect as in the KJ strain MGDG preparation can be expected also in these MGDG preparations. Incidentally, it is found that the fifth peak of the KJ strain MGDG preparation has high virucidal activity with respect to HSV-2 and IFV (Fig. 22).

### INDUSTRIAL APPLICABILITY

The anti-norovirus agent of the invention exhibits an action of promoting egestion/elimination of the virus from the body in addition to an action of suppressing norovirus proliferation, and is extremely effective in treatment of norovirus infectious diseases and prevention and inhibition of secondary infection. Meanwhile, the anti-norovirus agent of the invention can also be expected to have high safety since the effective ingredient thereof is a substance derived from a unicellular alga (preferably, a microalga).

This invention is not limited to the embodiments and the description of Examples described above. Various modifications are also included in this invention insofar as they are within the scope of the appended claims and they are easily reached by those skilled in the art. The entire contents of the articles, published unexamined patent applications, patent gazettes, and the like stated in the present specification are incorporated herein by reference.

## Claims

1. An anti-norovirus agent comprising a substance derived from a unicellular alga as an effective ingredient.

2. The anti-norovirus agent according to claim 1, wherein the unicellular alga is a microalga belonging to the genus Coccomyxa, Chlorella, Nannochloropsis, Arthrospira, or Euglena.

3. The anti-norovirus agent according to claim 1, wherein the unicellular alga is a microalga belonging to the genus Coccomyxa.

4. The anti-norovirus agent according to claim 3, wherein the microalga is a coccomyxa sp. KJ strain or a mutant strain thereof.

5. The anti-norovirus agent according to any one of claims 1 to 4, wherein the effective ingredient is a monogalactosyldiacylglycerol-containing extract of the unicellular alga.

6. The anti-norovirus agent according to claim 5, wherein a primary ingredient is monogalactosyldiacylglycerol.

7. The anti-norovirus agent according to claim 6, wherein the monogalactosyldiacylglycerol is one or more of monogalactosyldiacylglycerols selected from the group consisting of the following (1) to (6):
(1) monogalactosyldiacylglycerol in which a constituent fatty acid is C16:3 and C18:3;
(2) monogalactosyldiacylglycerol in which a constituent fatty acid is C16:2 and C18:3;
(3) monogalactosyldiacylglycerol in which a constituent fatty acid is C18:3 and C18:3;
(4) monogalactosyldiacylglycerol in which a constituent fatty acid is C16:2 and C18:2;
(5) monogalactosyldiacylglycerol in which a constituent fatty acid is C18:3 and C18:2; and
(6) monogalactosyldiacylglycerol in which a constituent fatty acid is C16:1 and C18:2.

8. The anti-norovirus agent according to any one of claims 5 to 7, wherein the effective ingredient is a substance obtained by subjecting an ethanol extract of the unicellular alga to chromatographic refinement and increasing a content of monogalactosyldiacylglycerol.

9. The anti-norovirus agent according to any one of claims 5 to 8, wherein a monogalactosyldiacylglycerol content in the effective ingredient is 70% (w/v) to 99% (w/v).

10. The anti-norovirus agent according to any one of claims 1 to 4, wherein the effective ingredient is an alga body.

11. The anti-norovirus agent according to any one of claims 1 to 4, wherein the effective ingredient is dry powder of an alga body.

12. An anti-norovirus agent comprising, as an effective ingredient, one or more of monogalactosyldiacylglycerols selected from the group consisting of the following (1) to (6) :
(1) monogalactosyldiacylglycerol in which a constituent fatty acid is C16:3 and C18:3;
(2) monogalactosyldiacylglycerol in which a constituent fatty acid is C16:2 and C18:3;
(3) monogalactosyldiacylglycerol in which a constituent fatty acid is C18:3 and C18:3;
(4) monogalactosyldiacylglycerol in which a constituent fatty acid is C16:2 and C18:2;
(5) monogalactosyldiacylglycerol in which a constituent fatty acid is C18:3 and C18:2; and
(6) monogalactosyldiacylglycerol in which a constituent fatty acid is C16:1 and C18:2.

13. The anti-norovirus agent according to any one of claims 1 to 12, wherein the anti-norovirus agent exhibits an action of suppressing proliferation of norovirus and/or an action of promoting egestion of the virus from the body.

14. A composition comprising the anti-norovirus agent according to any one of claims 1 to 13.

15. The composition according to claim 14, wherein the composition is a medicine for norovirus infectious diseases.

16. The composition according to claim 14, wherein the composition is food or feed.
